# FASCICULE DE BREVET EUROPEEN

(11) **EP 4 472 623 B1**
(45) Date de publication et mention de la délivrance du brevet: **31.12.2025**
(21) Numéro de dépôt: 23701973.2
(22) Date de dépôt: 27.01.2023
(51) Int. Cl.: A61K 31/198, A61K 31/455, A61K 31/51, A61K 31/525, A61K 31/714, A61P 25/02, A61P 39/00

(54) **NOUVELLE COMPOSITION COMPRENANT DE LA N-ACETYLCYSTEINE POUR PALLIER LES EFFETS INDESIRABLES D'UNE CHIMIOTHERAPIE**
NEUE ZUSAMMENSETZUNG MIT N-ACETYLCYSTEIN ZUR ÜBERWINDUNG DER NEBENWIRKUNGEN EINER CHEMOTHERAPIE
NOVEL COMPOSITION COMPRISING N-ACETYLCYSTEINE FOR OVERCOMING THE ADVERSE EFFECTS OF A CHEMOTHERAPY

(30) Priorité: 31.01.2022 FR 2200809
(43) Date de publication de la demande: 11.12.2024
(73) Titulaire: PRONUTRI, 06510 Carros (FR); UNIVERSITE COTE D'AZUR, 06100 Nice (FR); CENTRE NATIONAL DE LA RECHERCHE SCIENTIFIQUE, 75016 Paris (FR)
(72) Inventeur: COUNILLON, Laurent, 06100 NICE (FR); VERET, Amalric, 06510 CARROS (FR)
(74) Mandataire: Axe PI
(86) Numéro de dépôt international: PCT/EP2023/051988
(87) Numéro de publication internationale: WO 2023/144295

(56) Documents cités:
- WO-A1-00/76475
- CN-A- 113 693 231
- LIEBERMAN S ET AL: "Poly-MVA for treating non-small-cell lung cancer: A case study of an integrative approach", April 2006, ALTERNATIVE AND COMPLEMENTARY THERAPIES 200604 US, VOL. 12, NR. 2, PAGE(S) 77 - 80, ISSN: 1076-2809, XP009538216
- PENG-CHAN LIN ET AL: "N-acetylcysteine has neuroprotective effects against oxaliplatin-based adjuvant chemotherapy in colon cancer patients: preliminary data", SUPPORTIVE CARE IN CANCER, SPRINGER-VERLAG, DE, vol. 14, no. 5, 1 May 2006 (2006-05-01), pages 484 - 487, XP019349058, ISSN: 1433-7339, DOI: 10.1007/S00520-006-0018-9
- SCHLOSS JANET M ET AL: "A randomised, placebo-controlled trial assessing the efficacy of an oral B group vitamin in preventing the development of chemotherapy-induced peripheral neuropathy (CIPN)", SUPPORTIVE CARE IN CANCER, SPRINGER BERLIN HEIDELBERG, BERLIN/HEIDELBERG, vol. 25, no. 1, 9 September 2016 (2016-09-09), pages 195 - 204, XP036111819, ISSN: 0941-4355, [retrieved on 20160909], DOI: 10.1007/S00520-016-3404-Y

## Description

### DOMAINE TECHNIQUE

L'invention concerne une composition comprenant de la N-acétylcystéine (NAC) en combinaison avec d'autre(s) actif(s) pour son utilisation dans la prévention et/ou le traitement d'au moins un effet indésirable d'une chimiothérapie lors d'un traitement d'un cancer, en particulier la prévention et/ou le traitement des douleurs nociceptives et/ou neuropathiques chimio-induites.

### TECHNIQUE ANTERIEURE

La N-acétylcystéine ou NAC est un acide aminé non essentiel dérivé de la cystéine, avec un groupe acétyle qui est attaché à l'atome d'azote.

La NAC est donc un thiol qui peut s'oxyder en réduisant la quantité de radicaux libres. C'est également un précurseur du glutathion qui est un antioxydant bien connu.

Elle est connue pour être utilisée comme médicament et comme complément alimentaire.

La NAC est par exemple communément utilisée pour traiter les surdoses d'acétaminophène. La NAC peut également être utile dans d'autres conditions de stress oxydatif telles que l'apoptose, le dysfonctionnement mitochondrial, la neuroinflammation, ainsi que la dysrégulation du glutamate et de la dopamine.

En particulier, la NAC peut être utilisée pour améliorer les symptômes de plusieurs types de neuropathies induites par médicament.

Les neuropathies périphériques chimio-induites (CIPN) induites par exemple par les sels de platine, les taxanes, les alcaloïdes de la pervenche, le bortézomib, la thalidomide et l'éribuline sont un frein à leur utilisation.

En effet, ces effets indésirables, les plus souvent dose-dépendants et dose-limitants contraignent les cliniciens à diminuer la dose voir à stopper prématurément le traitement, compromettant ainsi les chances de guérison du patient. Le tableau clinique des CIPN, regroupe des symptômes sensoriels accompagnés ou non de symptômes moteurs et/ou autonomes pouvant être graves et invalidants. Les modalités d'apparition des CIPN sont aussi bien aiguë (oxaliplatine et paclitaxel) que chronique.

Les neuropathies induites par les agents anticancéreux peuvent apparaître immédiatement mais le plus souvent elles s'installent progressivement et peuvent persister plusieurs années après la fin des traitements, et les symptômes cliniques peuvent devenir irréversibles. Cela suggère la mise en place d'une série de mécanismes différents, qui peuvent aller d'actions immédiates à du remodelage et finalement à des dommages neuronaux.

Le tableau 1 ci-dessous fait un rappel rapide des différents types d'effets neuropathiques dont se plaignent les patients traités par des dérivés du platine ou par des taxanes.

**Tableau 1 : Effets secondaires des chimiothérapies observés en clinique**

| Agent anticancéreux | Effets sensoriels | Douleur | Effets moteurs | Effets autonomiques | Reflexes | Récupération |
|---|---|---|---|---|---|---|
| Cisplatine | Paresthésie, Vibration, Proprioception, Sensibilité, thermique aléatoire | Dysesthésie | Normal | Rare (dérégulation orthostatique | Réduits | |

| Carbolatine | Similaire au cisplatine | | | | | |
|---|---|---|---|---|---|---|
| Oxaliplatine (réaction aigüe) | | Dysesthésie, allodynie au froid, hyperalgésie mécanique | Crampes musculaires | Normal | Normal | Récupération après quelques jours |

| Oxaliplatine (chronique) | Similaire au cisplatine | | | | | |
|---|---|---|---|---|---|---|
| Paclitaxel, Docétaxel | Paresthésie, Vibration, Proprioception, Sensibilité, thermique et mécanique | Dysesthésie, sensation douloureuse de brûlure, sensibilité paradoxale au chaud | Rare (faiblesse proximale > distale) | Rare (dérégulation orthostatique) | Réduits | Généralement pas de récupération et progression possible après l'arrêt du traitement |

Pour les effets secondaires à court ou moyen terme, les stimuli mécaniques, thermiques ou douloureux sont dans tous les cas détectés et transmis par des canaux ioniques dont les principaux connus sont mentionnés dans le tableau 2 ci-dessous. Il a été montré que le cisplatine a un effet aigu sur certains de ces canaux (Milosavljevic et al., Cancer Res., 2011).

**Tableau 2: Exemples de canaux impliqués dans la nociception et la mécanosensation**

| **Canaux** | **Rôle** |
|---|---|
| **Canaux potassiques seuils** | |
| TREK1/TRAAK | Neuroprotection, douleur, dépression |
| TREK2 | Douleur polymodale/nociception, sensibilité au chaud et au froid, allodynie au froid associé à l'oxaliplatine |
| Kv1.1/1.2/1.4 | Neuropathies périphériques |

| **Canaux sodiques excitateurs** | |
|---|---|
| ASIC1a/ASIC2a/ASIC3 | Nociception lors des acidoses de tissu et inflammation |
| Nav1.7/Nav1.8/Nav1.9 | Allodynie et douleurs inflammatoires |

| **Autres canaux excitateurs** | |
|---|---|
| P2X2-3/3/P2X4 | Allodynie et douleurs inflammatoires |
| TRPV1/TRPM8/TRPA1 | Sensibilité thermique |
| Piezo 2 | Toucher fin et perception de la douleur |
| Caᵥa2δ-1 (Cav1.2/1.3/Cav2.1-2.3) | Sensibilité au toucher |

De nombreux traitements préventifs et curatifs ont été testés dans cette indication, à savoir les neuropathies chimio-induites, avec plus ou moins de succès (Thèse de Thibaut Fayolle, « Stratégie thérapeutique des neuropathies périphériques chimio-induites », UNIVERSITE CLERMONT AUVERGNE, 2018).

A titre d'exemples de composés utilisés dans la prévention et le traitement des neuropathies chimio-induites (Emilie Le Rhun, « Caractéristiques cliniques des neuropathies chimio-induites », CHRU Lille et Unicancer, 2016), on peut citer :
- parmi les composés ne semblant pas présenter d'intérêt préventif: acide lipoïque, nimodipine, gabapentine, lamotrigine, acetyl I-carnitine, venlafaxine ;
- parmi les composés présentant des résultats préventifs variables: amifostine, glutathion, infusions de calcium et magnésium, vitamine E ;
- parmi les composés présentant un intérêt préventif possible: N-acétylcystéine, carbamazepine et oxcarbazepine, Xaliprodenis (agoniste 5-hydroxytryptamine 1A), Goshajinkigan, érythropoïétine, PFT-µ, acide gras oméga-3, gel topique contenant du baclofène, amitriptyline, nortriptyline et kétamine, combinaison de Vitamine B12/B6 ;
- parmi les composés pharmaceutiques présentant un intérêt curatif éventuel : la duloxetine ;
- parmi les composés pharmaceutiques présentant un effet curatif discutable : la gabapentine ou pregabaline, la venlafaxine, amitriptyline, inhibiteurs recapture sérotonine et norépinéphrine, opïoides, anesthésiques locaux, perfusion de lidocaïne, dextromethorphane ;
- parmi les composés ne semblant pas présenter d'effet curatif : le lamotrigine, l'imipramine.

A cet effet, on connaît également le document WO03045334 qui décrit une composition chimioprotectrice (l'agent chimiothérapeutique étant par exemple du cisplatine) comprenant au moins deux chimioprotecteurs choisis dans le groupe consistant en méthionine, N-acétyl-DL-méthionine, S-adénosylméthionine, cystéine, homocystéine, cystathione, cystéamine, N-acétylcystéine, glutathion, glutathion éthylester, glutathion, glutathion diéthylester triéthylester, cystéamine, DiNAC, RibCys, RibCyst, ss-LactCys, a-LactCys, MeliCys, MaltCys, CellCys, OTCA, allopurinol, 1-méthylallopurinol, 2-méthylallopurinol, 5-méthylallopurinol 1,7-méthylallopurinol, 7-méthylallopurinol, 7-méthylallopurinol, 2,5-diméthylallopurinol, 1,7-diméthylallopurinol, 2,7-diméthylallopurinol, 5,7-diméthylallopurinol, 2,5,7-triméthylallopurinol, I-éthoxycarbonylallopurinol, l-éthoxycarbonyl-5-méthylallopurinol, 2-phényl-1, 2-benzoisosélénazol-3(2H)-one et 6-diSeCD.

De même, le document WO2007044700 concerne une combinaison thérapeutique comprenant deux agents protecteurs ou plus choisis dans un groupe constitué d'agents protecteurs de la méthionine spécifiques, la N-acétylcystéine, la carnitine, les ions magnésium, l'acide lipoïque, l'ebselen, le glutathion et l'ester de glutathion, administrée pour traiter l'ototoxicité, la néphrotoxicité, la neurotoxicité, l'alopécie, les troubles gastro-intestinaux ou la survie réduite chez un patient subissant un traitement avec une quantité efficace chimiothérapeutique d'un composé antitumoral de platine.

On connaît également le document WO0076475 qui décrit des compositions pour leur utilisation dans la prévention ou le traitement des effets indésirables d'une radiothérapie ou chimiothérapie, en particulier un exemple contenant explicitement 50 ppm de cyanocobalamine (vitamine B12) et 500 ppm de N-acetylcysteine, ainsi que 200 ppm de KCI et 500 ppm d'extrait de thé vert. Toutefois, une telle composition apparaît avoir un effet protecteur limité sur les neuropathies induites par la vincristine.

Le document CN113693231 décrit un complément alimentaire comprenant notamment de la γ-glutamylcysteine et des vitamines en particulier B1, B2, B6 et B12 pour assurer un apport nutritif et lutter contre les effets toxiques et secondaires provoqués par la radiothérapie et la chimiothérapie, avec un effet significatif sur les dommages de la muqueuse du tube digestif.

La publication Lieberman et al. « Poly-MVA for treating non-small-cell lung cancer: A case study of an integrative approach » (04/2006) décrit un patient traité par chimiothérapie (5FU et mitomycine C) pour un cancer du poumon et prenant un supplément comprenant de l'acetylcysteine, de la cyanocobalamine, de la thiamine et de la riboflavine.

La publication Peng-Chan Lin et al., Supportive Care in Cancer, Vol.14(5), mai 2006, pages 484-487, divulgue l'utilisation de la NAC dans le traitement des neuropathies chimio-induites.

Par ailleurs, le document US20020058628 divulgue un procédé de traitement d'une maladie ou d'un état pour lequel le traitement implique la promotion de la croissance des processus cellulaires neuronaux et dont la thérapie comprend l'administration d'un agoniste pour les récepteurs de surface des cellules neuronales qui favorisent la croissance des processus neuronaux, par exemple la neuropathie induite par la chimiothérapie, ledit procédé comprenant l'administration d'une quantité thérapeutiquement efficace dudit agoniste en combinaison avec une concentration thérapeutiquement efficace d'un ou plusieurs antioxydants/capteurs de radicaux libres et/ou d'un agent capable d'augmenter les taux de thiols intracellulaires et/ou d'un stéroïde. Un thiol intracellulaire représentatif est le glutathion, l'agent capable d'élever les taux de thiol intracellulaire est la N-acétylcystéine (NAC), et des antioxydants représentatifs piégeurs de radicaux libres peuvent être choisis dans le groupe constitué par la vitamine C, la vitamine E, leurs analogues et leurs mélanges. Un analogue représentatif de la vitamine E est le trolox.

Enfin, il est connu que la déficience en vitamine B12 peut conduire à des troubles neurologiques et comportementaux divers tels que l'ataxie, une faiblesse musculaire, une incontinence, une hypotension, des troubles de la vision, des troubles psychotiques et de l'humeur.

Toutefois, les données apparaissent être insuffisantes pour conclure que l'un des agents chimioprotecteurs potentiels tels que par exemple l'acétylcystéine, amifostine, calcium et magnésium, diéthyldithiocarbamate, glutathion, ORG 2766, oxcarbazépine, acide rétinoïque ou vitamine E, puisse prévenir ou limiter efficacement la neurotoxicité des médicaments à base de platine chez les patients humains, tel que cela a été déterminé à l'aide de mesures quantitatives objectives de la neuropathie (Albers JW et al., « Les interventions pour la prévention des lésions nerveuses causées par le cisplatine et d'autres médicaments anti-tumoraux à base de platine », Cochrane Database of Systematic Reviews 2014, Issue 3. Art. No.: CD005228. DOI: 10.1002/14651858.CD005228.pub4).

### PROBLEME TECHNIQUE

Considérant ce qui précède, un problème que se propose de résoudre la présente invention consiste à développer une nouvelle combinaison comprenant de la N-acétylcystéine (NAC) et présentant un effet potentialisé dans la prévention et/ou le traitement d'au moins un effet indésirable d'une chimiothérapie lors d'un traitement d'un cancer.

### SOLUTION APPORTEE

La solution à ce problème posé a pour premier objet une composition comprenant, dans un milieu physiologiquement acceptable, de la N-acétylcystéine (NAC), caractérisée en ce qu'elle comprend en outre de la Vitamine B12 à une concentration comprise entre 0,04 et 0,08% en poids du poids total de la composition et/ou une combinaison de Vitamines B1, B2 et B3, pour son utilisation dans la prévention et/ou le traitement des douleurs nociceptives et/ou neuropathiques chimio-induites.

De manière surprenante, parmi le nombre de combinaisons possibles d'agents chimioprotecteurs, et considérant les combinaisons déjà envisagées et testées et notamment avec la N-acétylcystéine (NAC), il n'est pas évident d'obtenir un effet potentialisé avec l'utilisation spécifique de N-acétylcystéine (NAC) combinée avec de la Vitamine B12 et/ou une combinaison de Vitamines B1, B2 et B3 pour pallier les effets indésirables d'une chimiothérapie.

### AVANTAGES APPORTES

Le Demandeur a notamment pu développer, de manière surprenante, une composition comprenant de la NAC en combinaison avec d'autre(s) actif(s) qui est particulièrement efficace dans la prévention et/ou le traitement d'au moins un effet indésirable d'une chimiothérapie lors d'un traitement d'un cancer, notamment dans la prévention et/ou le traitement des douleurs nociceptives et/ou neuropathiques chimio-induites.

La composition selon l'invention permet plus particulièrement de diminuer les doses utiles de NAC tout en gardant le même effet.

### BRÈVE DESCRIPTION DES DESSINS

L'invention et les avantages qui en découlent seront mieux compris à la lecture de la description et des modes de réalisation non limitatifs qui suivent, illustrés au regard des dessins annexés dans lesquels :
La figure 1 illustre l'effet des taxanes (A et C) et du cisplatine (B et D) sur l'expression de la sous-unité Cava2δ-1 (A et B) et le canal Piezo 2 (C et D), impliqués respectivement dans la transmission de la douleur et la détection des stimuli mécaniques.
La figure 2 illustre les Facteurs de transcription ciblant les gènes dont l'expression est modifiée après traitement aux taxanes et/ou le cisplatine platine dans les cultures primaires de DRG.
La figure 3 illustre la Cinétique unique de l'inhibition des gènes Piezo 2, CaVa2δ-1 et ARNT2 après 0, 4, 8 et 16h de traitement au cisplatine (1.25µg/ml) dans les DRG de souris issus de culture primaire.
La figure 4 illustre l'expression de Pizeo 2, Cav a2d1 et ARNT2 dans des neurones en culture primaire traités par un agent oxydant (Tert-Butyl hydroperoxide, TBHP).
La figure 5 illustre la dose réponse de la NAC sur l'expression génique d'Arnt2, Piezo 2 et CaVa2δ-1 après 24h d'exposition à 1.25µg/ml de cisplatine.
La figure 6 illustre la dose réponse du Beta Carotène sur l'expression génique d'Arnt2, Piezo 2 et CaVa2δ-1 après 24h d'exposition à 1.25µg/ml de cisplatine.
La figure 7 illustre la dose réponse de la Vitamine B12 sur l'expression génique d'Arnt2, Piezo 2 et CaVa2δ-1 après 24h d'exposition à 1.25µg/ml de cisplatine.
La figure 8 illustre la dose réponse de la Lutéoline sur l'expression génique d'Arnt2, Piezo 2 et CaVa2δ-1 après 24h d'exposition à 1.25µg/ml de cisplatine.
La figure 9 illustre l'effet de combinaisons de NAC et de différentes Vitamines sur l'expression génique d'Arnt2, Piezo 2 et CaVa2δ-1 après 24h d'exposition à 1.25µg/ml de cisplatine.
La figure 10 illustre l'effet de combinaisons de NAC et de Beta Carotène sur l'expression génique d'Arnt2, Piezo 2 et CaVa2δ-1 après 24h d'exposition à 1.25µg/ml de cisplatine.

### DESCRIPTION DES MODES DE REALISATION

Dans cette description, à moins qu'il ne soit spécifié autrement, il est entendu que, lorsqu'un intervalle est donné, il inclut les bornes supérieures et inférieure dudit intervalle.

L'invention concerne une composition comprenant, dans un milieu physiologiquement acceptable, de la N-acétylcystéine (NAC) pour son utilisation dans la prévention et/ou le traitement d'au moins un effet indésirable d'une chimiothérapie lors d'un traitement d'un cancer, et plus particulièrement des douleurs nociceptives et/ou neuropathiques chimio-induites.

La NAC est un acide aminé non essentiel dérivé de la cystéine, avec un groupe acétyle qui est attaché à l'atome d'azote.

La NAC est donc un promédicament de l'acide aminé endogène L-cystéine, un précurseur du glutathion et est connue pour posséder des activités mucolytiques, antioxydantes et potentiellement cytoprotectrices, anticancéreuses et anti-inflammatoires.

Lors de son administration, l'acétylcystéine exerce son activité mucolytique en réduisant les liaisons disulfure dans les mucoprotéines, entraînant la liquéfaction du mucus et réduisant sa viscosité.

Son activité antioxydante est attribuée à la capacité du GSH pour piéger les espèces réactives de l'oxygène (ROS), empêchant ainsi les dommages cellulaires induits par les ROS, diminuant le stress oxydatif, protégeant les cellules contre les effets néfastes des radicaux libres et empêchant l'apoptose dans ces cellules.

De plus, cela peut inhiber la prolifération, la progression et la survie des cellules tumorales, dans les cellules tumorales sensibles qui dépendent de la signalisation médiée par les ROS pour leur prolifération et leur comportement malin.

Dans certaines circonstances, l'acétylcystéine est capable d'induire l'apoptose dans les cellules sensibles, y compris certaines cellules tumorales, via la voie intrinsèque dépendante des mitochondries mais n'impliquant pas le stress du réticulum endoplasmique.

L'acétylcystéine possède également une activité anti-inflammatoire via la modulation de la voie du facteur nucléaire-kappa B (NF-kB) et la modulation de la synthèse des cytokines.

La composition selon l'invention se caractérise en ce qu'elle comprend en outre de la Vitamine B12 à une concentration comprise entre 0,04 et 0,08% en poids du poids total de la composition et/ou une combinaison de Vitamines B1, B2 et B3, préférentiellement de la Vitamine B12 à une concentration comprise entre 0,04 et 0,08% en poids du poids total de la composition prise seule ou en association avec la combinaison de Vitamines B1, B2 et B3.

La Vitamine B12, également appelé cobalamine, est une vitamine hydrosoluble essentielle très complexe qui contient le minéral cobalt. Cette vitamine est produite naturellement par les micro-organismes intestinaux et que l'on trouve également dans le sol et l'eau. Elle est nécessaire à la synthèse de l'ADN et à la production d'énergie cellulaire.

La vitamine B12 a de nombreuses formes, y compris les formes cyano, méthyl, désoxyadénosyl et hydroxy-cobalamine. La forme cyano est la forme la plus utilisée.

Avantageusement, la composition selon l'invention comprend en outre une combinaison de vitamines B1, B2 et B3, à des proportions différentes ou égales, préférentiellement à des proportions égales, prise seule ou préférentiellement en association avec la Vitamine B12.

La vitamine B1, également appelée la thiamine, est un précurseur métabolique de la thiamine pyrophosphate (TPP), une coenzyme essentielle à certaines décarboxylases. Elle est indispensable à la transformation des glucides en énergie par le cycle de Krebs et est nécessaire au bon fonctionnement du système nerveux et des muscles. Elle est en effet indispensable à la transformation du pyruvate produit par la glycolyse et toxique pour le système nerveux. Elle peut également inhiber l'action du glucose et de l'insuline dans la prolifération des cellules musculaires lisses artérielles. La thiamine peut également protéger contre la toxicité du plomb en inhibant la peroxydation lipidique induite par le plomb.

La vitamine B2 ou riboflavine, est une vitamine nécessaire à la synthèse de flavine adénine dinucléotide (FAD) et de la flavine mononucléotide (FMN), deux cofacteurs essentiels aux flavoprotéines. Ces cofacteurs sont d'une importance vitale dans la respiration normale des tissus, l'activation de la pyridoxine, la conversion du tryptophane en niacine, le métabolisme des lipides, des glucides et des protéines, et la détoxification médiée par la glutathion-réductase.

La riboflavine peut également être impliquée dans le maintien de l'intégrité des érythrocytes. De plus, elle est essentielle pour une peau, des ongles et des cheveux sains.

La niacine, également connue sous le nom d'acide nicotinique et de vitamine B3, est une vitamine B essentielle hydrosoluble qui, lorsqu'elle est administrée à fortes doses, est efficace pour abaisser le taux de cholestérol des lipoprotéines de faible densité (LDL) et augmenter le taux de cholestérol des lipoprotéines de haute densité (HDL), ce qui confère à cet agent une valeur unique dans le traitement de la dyslipidémie.

La composition selon l'invention est utilisée dans la prévention et/ou le traitement d'au moins un effet indésirable d'une chimiothérapie lors d'un traitement d'un cancer, et plus particulièrement des douleurs nociceptives et/ou neuropathiques chimio-induites.

Le cancer est une maladie caractérisée par la prolifération incontrôlée des cellules, lié à l'échappement aux mécanismes de régulation qui assure le développement harmonieux de l'organisme.

Le cancer démarre par une cellule normale qui se transforme et devient anormale. Cette transformation peut être due à : une activation constante des proto-oncogènes, une inactivation des gènes suppresseurs de tumeurs ou encore à l'inactivation des gènes réparateurs de l'ADN.

Les cancers plus particulièrement ciblés selon l'invention résultent de tumeurs solides et sont choisis préférentiellement parmi les cancers du testicule, de la prostate, de l'ovaire, du col de l'utérus, du sein, de la vessie, les tumeurs ORL, de la tête et du cou, de l'œsophage, du foie, du poumon, les tumeurs cérébrales, ou encore les neuroblastomes.

Il existe aujourd'hui plusieurs thérapies anticancéreuses : le traitement chirurgical, la radiothérapie et la chimiothérapie.

La chimiothérapie est un traitement pharmacologique qui va permettre de cibler les voies de signalisation ou encore le micro-environnement tumoral. C'est le traitement le plus utilisé pour les cancers métastasés. Elle peut être combinée avec d'autres thérapies. La chimiothérapie va permettre de cibler la division cellulaire et donc le cycle cellulaire : certaines molécules du traitement vont être des intercalants de l'ADN, d'autres vont jouer sur des enzymes qui contrôlent la topologie de l'ADN ce qui va perturber la réplication de l'ADN, certaines molécules vont venir cibler les microtubules, et enfin d'autres molécules vont faire des adduits entre les 2 brins d'ADN, ce qui va être détecté comme des lésions de cette molécule et entraîner ainsi la mort cellulaire par différentes voies de signalisation.

Cependant, la chimiothérapie présente certaines limites. En effet, elle va également cibler les cellules normales et donc présenter une toxicité envers ces cellules.

Un milieu physiologiquement acceptable désigne un milieu compatible et adapté pour une utilisation en contact avec des cellules humaines et animales, en particulier avec la peau, les muqueuses et/ou les phanères, sans toxicité, irritation, réponse allergique indue et similaires, et proportionné à un rapport avantage/risque raisonnable.

Un milieu physiologiquement acceptable selon l'invention peut comprendre tout excipient connu et utilisé dans le domaine pharmaceutique et des compléments alimentaires, compatible avec la NAC et Vitamines B12 et/ou B1, B2 et B3 utilisées selon l'invention.

A titre d'exemple non limitatif on peut citer des solvants, tampons, aromatisants, liants, agents chélatants, agents tensioactifs, épaississants, humectants, hydratants, conservateurs, antioxydants, agents apaisants, colorants, parfums et similaires, ou leur mélange.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée. Leur concentration est également choisie de sorte à ce qu'elle ne nuise pas aux propriétés avantageuses des composés utilisés selon l'invention.

A titre d'exemple d'excipients, on peut citer notamment le xylitol, gomme laque, lactose, sorbitol, saccharose, amidon de blé, stéarate de magnésium, l'acide lipoïque, ou encore des huiles riches en oméga 3 telles que l'huile de lin, l'huile de périlla, l'huile de chanvre, l'huile de cameline, l'huile d'Inca inchi, l'huile de noix, l'huile de colza, l'huile de chia, l'huile de rose musquée du Chili, l'huile d'argousier.

La composition selon l'invention est préférentiellement utilisée pour la prévention et/ou le traitement des douleurs nociceptives et/ou neuropathiques chimio-induites, plus préférentiellement lorsque la chimiothérapie est induite par les dérivés de platine choisis parmi le cisplatine, l'oxiplatine et le carboplatine, ou par les taxanes choisis parmi le paclitaxel et le docétaxel.

Les taxanes et dérives de platine sont utilisés, en première ligne ou en combinaison, dans plus de 80% des cancers traités par chimiothérapie. Ils permettent de traiter les cancers de l'ovaire, du sein, du foie, du poumon, de la tête et du cou, du testicule, de la prostate et de l'œsophage.

Les taxanes sont une classe de diterpènes. Le principal mécanisme d'action de la classe des taxanes est l'inhibition de la fonction des microtubules. Les microtubules sont essentiels pour la division cellulaire, et les taxanes bloques la division normale des cellules. Les taxanes jouent ainsi le rôle de poison du fuseau mitotique. Ils sont également supposés être radio-sensibilisants.

Cette classe comprend le paclitaxel connu sous le nom commercial de Taxol. Le paclitaxel est une molécule produite par des champignons endophytes, que l'on trouve également dans des extraits de certaines espèces d'ifs à qui elle confère leur forte toxicité.

Le paclitaxel va inhiber la dépolymérisation des microtubules, bloquant ainsi le mécanisme de la mitose. C'est une molécule médicamenteuse utilisée dans le traitement des cancers et injectée par perfusion. En cancérologie, le paclitaxel est principalement utilisé dans le cancer du poumon, le cancer du sein et le cancer de l'ovaire.

Les effets secondaires sont ceux rencontrés avec les traitements anticancéreux : une diminution des globules blancs, des plaquettes et des globules rouges, perte de cheveux et inflammation des muqueuses, en particulier de la bouche. Les effets secondaires spécifiques du paclitaxel sont une atteinte des nerfs périphériques, parfois sévère, un risque de rétention hydrique (ascite, épanchements pleuraux ou péricardiques), des réactions cutanées, une altération des ongles et des réactions d'hypersensibilité à l'injection.

Le docétaxel est un alcaloïde obtenu par hémisynthèse à partir d'une molécule extraite de l'if européen. Le docétaxel, est un analogue du paclitaxel, de structure voisine mais avec une activité doublée. Il en diffère surtout par sa toxicité et son efficacité antitumorale. Le docétaxel stabilise également les microtubules en inhibant leur dépolymérisation par liaison stable à la tubuline et entraîne un blocage de la mitose. Il est injecté par voie intra-veineuse.

L'utilisation de docétaxel entraîne une toxicité au niveau de la moëlle osseuse, l'apparition d'œdèmes et épanchements par rétention d'eau et des troubles neurologiques. Ceci-dit il est efficace pour d'assez nombreuses tumeurs.

Les sels de platine sont une classe de médicaments de chimiothérapie à base de platine. Cette classe regroupe trois molécules couramment utilisées : le cisplatine, l'oxaliplatine, et le carboplatine. Ils sont souvent mentionnés comme alkylants mais font essentiellement des adduits guanine-guanine dans la double hélice d'ADN pour former le complexe ADN-platine, qui va bloquer la réplication, la transcription et la réparation de l'ADN. Ils ont également des propriétés immunosuppressives.

Les sels de platine sont des produits cytotoxiques puissants. Ainsi, ils provoquent :
- une néphrotoxicité qui est une nécrose tubulaire aigüe pouvant aboutir à une insuffisance rénale chronique ;
- une ototoxicité, avec une éventuelle perte définitive d'audition dans les hautes fréquences ;
- des troubles de la vision ;
- des troubles du goût ;
- une neurotoxicité : neuropathies périphériques ;
- une neutropénie ;
- une myélosuppression ;
- des nausées et vomissements fréquents ; et
- des réactions allergiques.

Selon l'invention, le terme « traitement » désigne une amélioration, une prophylaxie ou un renversement d'une maladie ou d'un trouble, ou d'au moins un symptôme discernable de celui-ci. Il s'agit également d'une amélioration, d'une prophylaxie ou d'un renversement d'au moins un paramètre physique mesurable lié à la maladie ou au trouble traité, ce qui n'est pas nécessairement perceptible par le sujet. Dans un autre mode de réalisation, le terme « traitement » désigne l'inhibition ou le ralentissement de la progression d'une maladie ou d'un trouble, soit physiquement, par exemple, la stabilisation d'un symptôme discernable, physiologiquement, par exemple, la stabilisation d'un paramètre physique, ou les deux. Le terme « traitement » désigne également le retard de l'apparition d'une maladie ou d'un trouble. Dans certains modes de réalisation particuliers de l'invention, la composition d'intérêt est administrée en tant que mesure préventive. Dans ce contexte, le terme « prévention » désigne une réduction du risque d'acquisition d'une maladie ou d'un trouble spécifié.

Plus particulièrement, le demandeur a pu mettre en évidence que la composition utilisée selon l'invention est particulièrement efficace pour la prévention et/ou le traitement des douleurs nociceptives et/ou neuropathiques chimio-induites.

Les douleurs nociceptives résultent de l'activation du système de la nociception par une lésion interne ou externe de l'organisme. En oncologie, ceci explique plus de 70% des syndromes douloureux. La lésion peut être causée par le cancer comme par les traitements du cancer.

Les douleurs neuropathiques sont la conséquence d'une lésion du système nerveux : compression d'un nerf par la tumeur, toxicité neurologique d'une chimiothérapie, séquelle d'une chirurgie par exemple. Elles sont ressenties de manière inhabituelle : brûlure, fourmillement, décharges électriques. Quand la cause de la souffrance neurologique disparaît, la douleur peut persister de manière transitoire ou chronique (séquelle douloureuse). Leur diagnostic est parfois difficile, car ces douleurs peuvent survenir avec un décalage dans le temps après la lésion nerveuse. En oncologie, les douleurs neuropathiques sont retrouvées, seules ou en association avec une douleur nociceptive.

Les différents types d'effets neuropathiques dont se plaignent les patients traités par les dérivés du platine ou par les taxanes sont décrits dans le tableau 1 ci-avant.

Les canaux impliqués dans la nociception et la mécano sensation sont quant à eux décrits dans le tableau 2 ci-avant. Le demandeur a pu montrer que le stress oxydatif induit par ces agents anticancéreux perturbe fortement l'expression de certains de ces canaux, sur culture de neurones sensoriels. Il a également montré que l'ajout d'un antioxydant puissant et parfaitement bien toléré chez l'homme, le NAC, limite fortement cette perturbation dans l'expression et supprime les neuropathies qui en découlent sur un modèle murin.

Comme illustré dans l'exemple 5, le demandeur a pu démontrer qu'il existe une synergie entre NAC et la vitamine B12 à une concentration comprise entre 0,04 et 0,08% en poids du poids total de la composition, puisque cette dernière permet de baisser les doses utiles de NAC en gardant le même effet.

La composition utilisée selon l'invention comprend la NAC à une concentration comprise entre 10 et 90%, par exemple 15%, 20%, 25%, 30%, 35%, 40%, 45%, 50%, 55%, 60%, 65%, 70%, 75%, 80%, 85%, préférentiellement entre 20 et 80%, plus préférentiellement entre 25 et 75%, par exemple 25% et 75%, en poids du poids total de la composition.

La composition utilisée selon l'invention comprend la vitamine B12 à une concentration comprise entre 0,04 et 0,08 %, par exemple 0,05%, 0,06%, 0,07%, préférentiellement entre 0,05 et 0,07%, par exemple 0,06%, en poids du poids total de la composition, prise seule ou en association avec une combinaison de Vitamines B1, B2 et B3.

Selon un autre mode de réalisation, la composition utilisée selon l'invention comprend une combinaison de Vitamines B1, B2 et B3, à des proportions différentes ou égales, préférentiellement à des proportions égales, à une concentration totale comprise entre 20 et 90%, par exemple 15%, 20%, 25%, 30%, 35%, 40%, 45%, 50%, 55%, 60%, 65%, 70%, 75%, 80%, 85%, préférentiellement entre 40 et 60%, par exemple 50%, en poids du poids total de la composition, prise seule ou en association avec de la Vitamine B12, préférentiellement en association avec de la Vitamine B12 à une concentration comprise entre 0,04 et 0,08 %, plus préférentiellement entre 0,05 et 0,07% en poids du poids total de la composition.

Avantageusement, selon un premier mode de réalisation, la composition utilisée selon l'invention comprend :
- entre 50 et 90% de NAC en poids du poids total de la composition ; et
- entre 0,04 et 0,08% de Vitamine B12 en poids du poids total de la composition.

De façon particulièrement avantageuse, la composition utilisée selon l'invention comprend :
- entre 70 et 80% de NAC en poids du poids total de la composition ; et
- entre 0,05 et 0,07% de Vitamine B12 en poids du poids total de la composition.

De façon alternative, selon un autre mode de réalisation, la composition utilisée selon l'invention comprend :
- entre 10 et 30% de NAC en poids du poids total de la composition ; et
- entre 20 et 90% d'une combinaison de Vitamines B1, B2 et B3 en poids du poids total de la composition.

De façon avantageuse, la composition utilisée selon l'invention comprend :
- entre 15 et 30% de NAC en poids du poids total de la composition ; et
- entre 30 et 60% d'une combinaison de Vitamines B1, B2 et B3 en poids du poids total de la composition.

De façon alternative, selon un autre mode de réalisation, la composition utilisée selon l'invention comprend :
- entre 10 et 90% de NAC en poids du poids total de la composition ; et
- entre 0,04 et 0,08% de Vitamine B12 et entre 20 et 90% d'une combinaison de Vitamines B1, B2 et B3 en poids du poids total de la composition.

Avantageusement, la composition utilisée selon l'invention comprend :
- entre 15 et 30% de NAC en poids du poids total de la composition ; et
- entre 0,05 et 0,07% de Vitamine B12 et entre 30 et 60% d'une combinaison de Vitamines B1, B2 et B3 en poids du poids total de la composition.

De préférence, la dose journalière de NAC de la composition utilisée selon la présente invention est comprise entre 200 et 1200 mg et la dose journalière de vitamine B12 est comprise entre 0,16 et 1 mg et/ou la dose journalière de la combinaison de Vitamines B1, B2 et B3 est comprise entre 400 et 2400 mg, plus préférentiellement entre 280 et 1200 mg de NAC, entre 0,50 et 1 mg de vitamine B12 et/ou entre 560 et 2400 mg de la combinaison de Vitamines B1, B2 et B3.

Cette dose journalière est préférentiellement administrée en 1, 2 ou 3 prises (matin, midi et/ou soir) par exemple sous forme de 1, 2, 3, 4, 6, 7, 8, 9, 10, 11 ou 12 comprimés.

La composition utilisée selon l'invention est administrable par voie orale, injectable ou encore transdermique, préférentiellement par voie orale.

De manière avantageuse, la composition utilisée selon l'invention est administrable par voie orale, en une ou plusieurs formulations identiques ou différentes. Elle se présente sous une forme galénique quelconque normalement utilisée pour une administration orale et notamment sous la forme de gélule, comprimé, capsule, capsule molle, dragée, sachet, tube, flacon, chewing-gum, bille, émulsion, suspension, liquide, solution, ampoule, boisson, sirop, poudre par exemple contenue dans des sachets notamment des sachets alu-alu, solide, gel mou, semi-solide.

Avantageusement, la composition utilisée selon l'invention est formulée sous forme de comprimé, gélule, capsule, gel mou, semi-solide, solide, liquide ou poudre.

Encore plus avantageusement, la composition utilisée selon l'invention est formulée sous forme de microgranules à libération contrôlée en gélule.

De manière avantageuse, les microgranules sont enrobées d'une membrane semi-perméable qui protège les substances actives contre la dégradation et contrôle ainsi leur libération. Cette membrane va donc permettre de masquer le goût et/ou l'odeur des substances qu'elle enrobe, la stabilité des substances et la protection des actifs contre l'acidité au niveau de l'estomac. Elle va également permettre un contrôle de la libération des actifs.

Les microgranules sont préférentiellement sous forme de microgranules de xilitol et d'un mélange de plantes permettant ainsi une meilleure assimilation par l'organisme.

Un exemple de procédé de fabrication de ces microgranules est décrit dans l'exemple 8.

A titre d'exemple non limitatif, le poids final de la gélule est préférentiellement compris entre 200 et 600 mg. Plus préférentiellement encore, il est compris entre 300 et 500 mg. De façon particulièrement avantageuse, le poids final de la gélule est de 400 mg.

Selon un autre mode de réalisation, la composition utilisée selon l'invention est administrable par voie injectable sous forme liquide par exemple comme adjuvant dans une solution de chimiothérapie.

Selon un autre mode de réalisation, la composition utilisée selon l'invention est administrable par voie transdermique par exemple sous forme de patch ou de dispositif médical apte à augmenter la pénétration à travers la peau pour atteindre la circulation sanguine sans injection, avantageusement avec une libération prolongée et continue.

L'invention a aussi pour objet un produit comprenant :
- à titre de premier principe actif de la NAC, et
- à titre de deuxième principe actif de la vitamine B12 à une concentration comprise entre 0,04 et 0,08% en poids du poids total de la composition et/ou une combinaison de vitamines B1, B2 et B3,
comme produit de combinaison pour une utilisation simultanée, séparée ou étalée dans le temps pour son utilisation dans la prévention et/ou le traitement des douleurs nociceptives et/ou neuropathiques chimio-induites.

Selon un mode de réalisation, l'invention concerne un produit comprenant :
- à titre de premier principe actif de la NAC,
- à titre de deuxième principe actif de la vitamine B12 à une concentration comprise entre 0,04 et 0,08% en poids du poids total de la composition, et
comme produit de combinaison pour une utilisation simultanée, séparée ou étalée dans le temps pour son utilisation dans la prévention et/ou le traitement des douleurs nociceptives et/ou neuropathiques chimio-induites.

De façon alternative, l'invention concerne un produit comprenant :
- à titre de premier principe actif de la NAC, et
- à titre de deuxième principe actif une combinaison de vitamines B1, B2 et B3, comme produit de combinaison pour une utilisation simultanée, séparée ou étalée dans le temps pour son utilisation dans la prévention et/ou le traitement des douleurs nociceptives et/ou neuropathiques chimio-induites.

Selon un autre mode de réalisation, l'invention concerne un produit comprenant :
- à titre de premier principe actif de la NAC,
- à titre de deuxième principe actif de la vitamine B12 à une concentration comprise entre 0,04 et 0,08% en poids du poids total de la composition, et
- à titre de troisième principe actif une combinaison de vitamines B1, B2 et B3, comme produit de combinaison pour une utilisation simultanée, séparée ou étalée dans le temps pour son utilisation dans la prévention et/ou le traitement des douleurs nociceptives et/ou neuropathiques chimio-induites.

### EXEMPLES

### Exemple 1 : Identification de 2 gènes dont la modification d'expression est commune aux différents agents utilisés

Les inventeurs ont cherché à évaluer la façon dont les effets secondaires neuropathiques commencent à s'installer à moyen terme (effets sur lesquels il est le plus intéressant d'agir).

Les inventeurs ont plus particulièrement travaillé sur les variations d'expression du niveau d'expression des canaux qui sont les plus susceptibles d'expliquer ce qui se passe dans cette fenêtre de temps.

Du point de vue du mécanisme, il est clair que le remodelage de l'expression de canaux différents selon l'agent utilisé en chimiothérapie peut donner des symptômes similaires.

L'objectif est d'identifier des gènes dont le remodelage est similaire dans les chimiothérapies au platine ou aux taxanes.

### Matériel et Méthode

### 1. Culture primaire des ganglions rachidiens dorsaux (DRG) de souris

### Modèle : C57BI6 femelles de 8 à 15 semaines (Jeunes souris adultes qui ont atteint la maturité sexuelle)

Il a été choisi de travailler sur des souris femelles car l'apparition des neuropathies périphériques chimio-induites n'est pas liée au sexe de la souris ou du patient.

24H avant la dissection :
- Préparation des solutions
   i. HBSS-GLUCOSE :
      50 mL de HBSS 10X
      1.7 mL HEPES 1.5M
      10 mL de D-Glucose 0.5M
      5 mL de Peni-Streptomycine
      QSP à 100mL H2O miliQ
      Ajuster PH à 7.5 avec NaOH
      QSP 500mL H2O miliQ, Filtrer et mettre en bouteille (conserver à 4°C).
   ii. Milieu Neurobasal avec NGF :
      Pour 25mL de milieu : 500µL de B27, 250µL de péni-streptomycine, 250µL de L-Glutamine, 6.25µL de NGF (2000µg/mL) et compléter à 25mL avec du milieu Neurobasal.
   iii. Digestion enzymatique :
      Dans 4 mL de HBSS-GLUCOSE, mettre 80mg de Collagénase II et 200mg de Dispase, puis aliquoter dans des tubes de 2mL 200µL par tube et congeler.
- Coating plaque 12 puits

24 heures avant la dissection il faut faire un coating d'une plaque 12 puits.

Pendant 2h à température ambiante mettre par puit 500µL de PLL (Poly-L-Lysine) à 100µg/mL ; puis mettre 500µL de Laminin à 20µg/mL pendant 1h à 37°C.

Rincer avec du HBSS 1X et laisser sécher sous hôte jusqu'au lendemain.

### - Dissection de la souris

La dissection et l'extraction des DRG ne doivent pas dépasser 1h afin de limiter les dommages cellulaires. La dissection de la souris se fait en ventral et la colonne est extraite puis placée sous loupe binoculaire afin d'y extraire les DRG.

L'extraction a lieu à « sec » (pas dans du PBS froid), la colonne et les DRG sont souvent arrosés de HBSS-glucose froid afin de ne pas les laisser sécher. Après avoir récupéré entre 20-30 DRG dans un tube 2mL placés dans la glace, ils sont replacés sous loupe binoculaire afin de couper les filaments à l'aide d'un scalpel (on ne garde que les amas de neurones).

Les DRG sont ensuite récoltés dans un tube 15mL placés dans la glace le temps d'aller en culture cellulaire.

### - Digestion enzymatique des DRG

Les DRG sont rincés deux fois avec du HBSS-Glucose à température ambiante.

On récupère un tube de 2mL contenant le mélange enzymatique de Dispase et de Collagénase Il auquel on ajoute 50µL de CaCl2 à 0.2M.

On ajoute ensuite 1750µL de HBBS-Glucose.

Mettre 1mL de la digestion enzymatique sur les DRG et mettre à 37°C pendant 20minutes.

Mettre à nouveau 1mL de digestion enzymatique sur les DRG et mettre à 37°C pendant 20minutes.

Pendant le temps de digestion enzymatique, coater 3 seringues : 21G, 22G et 26G avec du SVF (Sérum Veau Foetal).

Une fois la digestion enzymatique terminée, rincer deux fois avec du HBSS-Glucose, rincer une fois avec 500µL du milieu Neurobasal avec NGF.

Mettre la quantité de milieu Neurobasal avec NGF nécessaire pour les puits (200µL/puits).

Passer les DRG dans les Seringues du 21G à 26G, 6 passages à chaque fois.

Mettre ensuite 200µL par puits et mettre à 37°C pendant 1heure.

Compléter avec 600µL de milieu Neurobasal avec NGF. Mettre à 37°C.

### 2.Traitement des DRG en culture

Le lendemain matin, déposer 1,25µg/ml de cisplatine avec les autres molécules à tester.

Traitement des DRG pendant 24 heures.

### Extraction d'ARN et RT-qPCR

### 3. QPCR

### - Extraction d'ARN

Les ARN sont extraits avec le kit « NucleoSpin RNA » de chez Macherey-Nagel.

Les ARN sont ensuite dosés au Nanodrop puis conservés à -80°C.

### - Reverse-transcription

Les reverse-transcriptions ont été faites en suivant le protocole du kit « SuperScript III Reverse Transcriptase » de chez Thermo Fisher Scientific en doublant les quantités d'ARN et de produits utilisés afin d'obtenir une plus grande concentration d'ADNc.

### - qPCR

Les qPCR ont été réalisées avec l'ADNc obtenu par reverse-transcription sans dilution en utilisant 4.625µL d'ADNc et 5.375µL de Mix PCR SyberROX (10µL final par puit).

Chaque puit a été fait en duplica.

### - Analyse des résultats

On considère que lorsque le seuil de cycle (CT ou Cycle Threshold) > 35, le gène n'est pas exprimé dans les cellules analysées.

Pour comparer nos résultats avec ou sans traitement, nous avons calculé le delta entre le gène cible et le gène de référence (36B4) pour chaque condition (ex. dCT_{PIEZO2} = CT_{PIEZO2} - CT_{36B4} = 27,4-21,6 = 5,8 (contrôle) ; 30,6-23,4 = 7,2 (cisplatine)).

Puis, nous avons fait le delta delta CT du gène cible pour la condition test et la condition contrôle (ex. ddCT _{PIEZO2} = dCT _{PIEZO2(cisplatine)} - dCT _{TREK1(contrôle)} = 7,2-5,8 = 1,4).

Enfin, nous avons déterminé l'expression relative en calculant : 2^{^-ddCT} (ex. pour PIEZO2 : 2^{^-ddCT} = 2^{^-1.4} = 0,40).

Dans notre exemple, l'expression relative de PIEZO2 dans les cellules traitées au cisplatine est donc de 0,40 par rapport aux cellules contrôles dont l'expression est normalisée à 1.

### - Microscopie

Sur les expériences réalisées et exemplifiées ci-après, une photo en microscopie (non illustrée) en contraste de phase est prise systématiquement pour visualiser l'effet du traitement en chimiothérapie et l'éventuel effet neuroprotecteur des molécules testées.

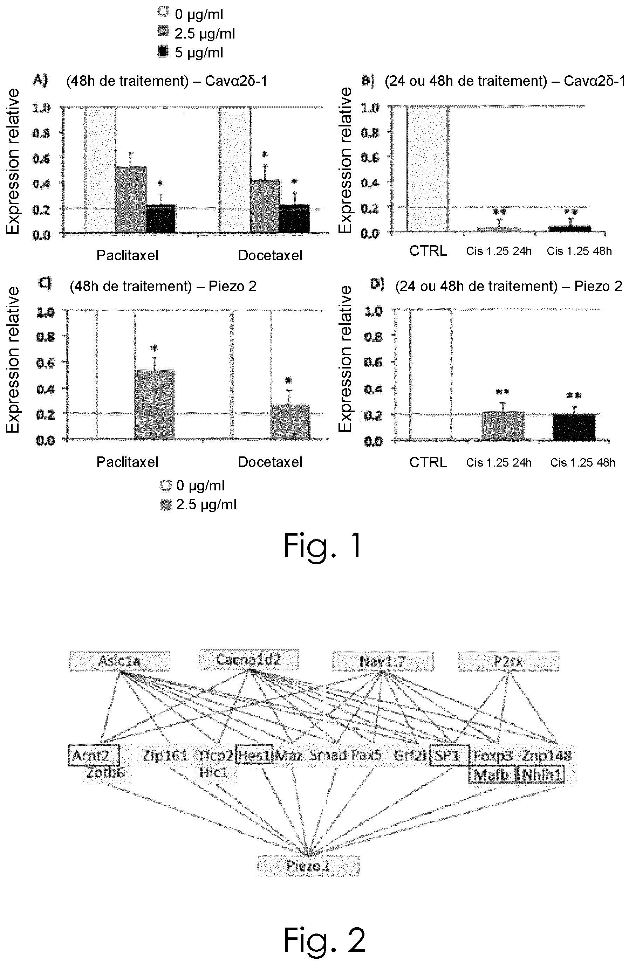

Dans cet exemple sur le remodelage génique induit par le cisplatine et les taxanes au niveau de canaux ioniques exprimés dans les DRG de souris issus de culture primaire, d'après les résultats obtenus et illustrés à la Figure 1, 2 gènes, à savoir Piezo 2 et CaVa2δ-1, pour lesquels la modification d'expression est commune aux différents agents utilisés, ont été plus particulièrement identifiés.

Les 2 gènes apparaissent donc présenter une réponse commune à un traitement par les dérivés du platine et les taxanes.

L'expression de Piezo 2 a donc notamment été utilisée comme un rapporteur intéressant pour tester des molécules chimio-protectrices.

### Exemple 2 : Identification de régulateurs en amont

En faisant une analyse des promoteurs des gènes dont on voit des modifications d'expression similaires, on a pu identifier un certain nombre de facteurs de transcription pouvant être des régulateurs communs tel qu'illustré à la Figure 2. Bien entendu il faut ensuite les tester en parallèle en QPCR.

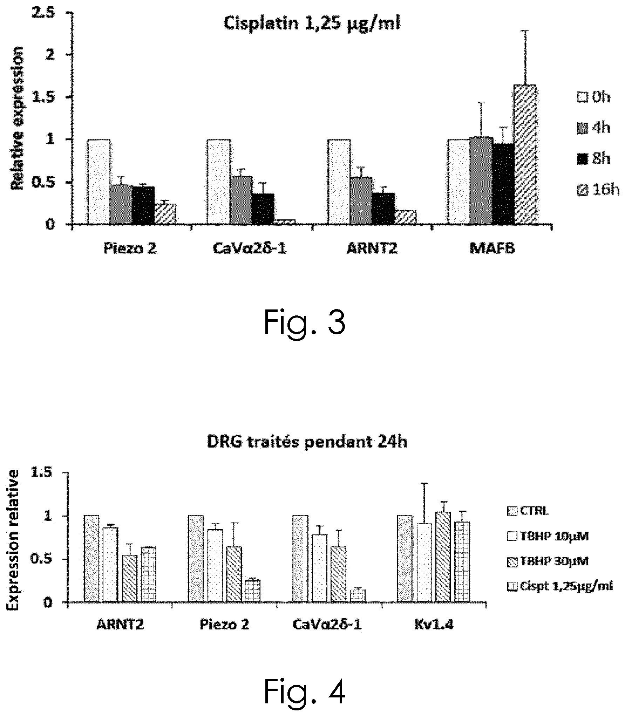

Tel qu'illustré par la Figure 3 relative à un de traitement au cisplatine (1.25µg/ml) dans les DRG de souris issus de culture primaire, le facteur de transcription qui a un pattern similaire à Piezo et CaVa2δ-1 est ARNT2.

Ceci est intéressant car il est au niveau protéique à la fois activé en réponse au stress oxydatif et les xénobiotiques et va à son tour activer des gènes de réponse à ces stress.

Cela a amené à faire l'hypothèse que le stress oxydatif induit par les chimiothérapies testées a un effet délétère à moyen terme sur l'expression d'ARNT2 ce qui amplifie leur effet toxique dans les neurones.

En revanche, tel qu'illustré par la Figure 3, les effets observés pour Mafb ne sont pas significatifs. L'expression des ARNm a été mesurée par qPCR et normalisée avec le gène de référence 36B4. Les résultats correspondent à la moyenne ± SEM.

### Exemple 3 : Test de l'effet d'un stress oxydatif

L'expression de Pizeo 2, CaVa2δ-1 et ARNT2 a été mesurée dans des neurones en culture primaire traités par un agent oxydant (Tert-Butyl hydroperoxide, TBHP).

L'expression de Kv1.4 (contrôle négatif) n'est pas impactée.

Les DRG de souris issus de culture primaire ont été traités au TBHP à 10 ou 30 µM ou à 1.25µg/ml de cisplatine (contrôle positif) versus contrôle (CTRL). L'expression des ARNm a été mesurée par qPCR et normalisée avec le gène de référence 36B4. Les résultats correspondent à la moyenne ± SEM.

Cet exemple est important car il montre bien, tel qu'illustré par la Figure 4, que l'on a un effet similaire entre les chimiothérapies et un agent produisant de la peroxydation.

Cela a conduit à tester des agents antioxydants sur l'expression des gènes rapporteurs identifiés, le plus évident étant la NAC.

### Exemple 4 : Test de l'effet de différentes molécules prises seules avec le cisplatine

L'effet de différentes molécules a été testée sur l'expression des mêmes gènes rapporteurs Pizeo 2, CaVa2δ-1 et ARNT2 en QPCR sur neurones de DRG en culture primaire :
- NAC
- Beta Carotène (provitamine A)
- Vitamine B12
- Lutéoline (Flavone)

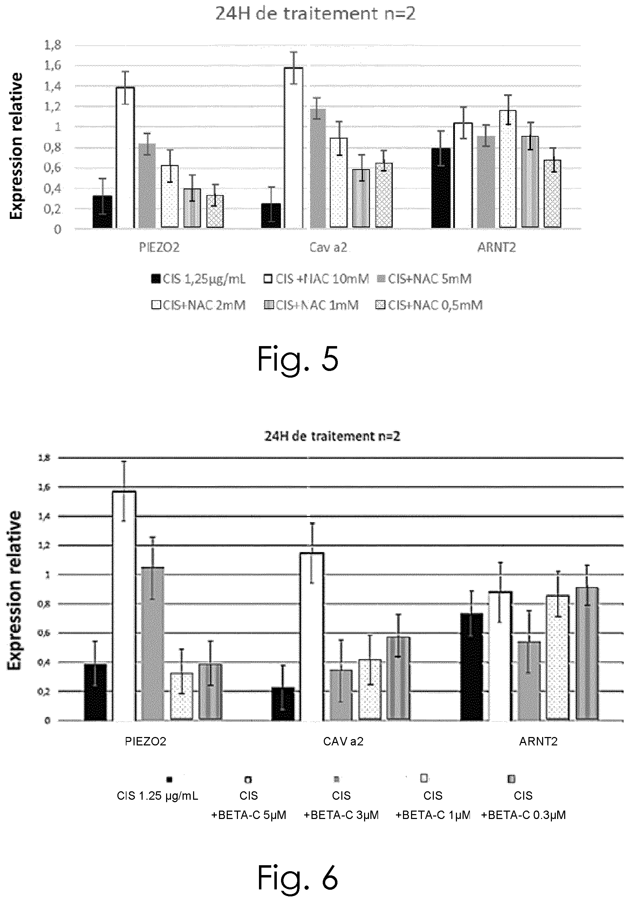

D'après les résultats obtenus, la NAC apparaît avoir un effet protecteur sur la morphologie des neurones de DRG, et permettre de faire remonter ex vivo les niveaux d'expression de Piezo2, CaVa2δ-1 et ARNT2 dans les neurones en culture primaire (tel qu'illustré à la Figure 5) mais aussi *in vivo* sur l'animal exposé à un protocole de chimiothérapie (non illustré). Enfin l'administration de NAC apparaît diminuer de façon spectaculaire l'hypersensibilité tactile (en réponse aux stimuli mécaniques induits par le test de Von Frey) déclenchée par le cisplatine et le paclitaxel (non illustré).

D'après les résultats obtenus et tel qu'illustré par la Figure 6, le Beta Carotène apparaît avoir un effet seulement à une dose 5µM, et pas aux doses inférieures testées (0,3, 1 et 3 µM). Bien qu'une telle dose de 5 µM soit une dose 1000 fois plus faible que celle utile pour la NAC, une telle dose s'avère être un peu toxique pour les neurones en culture (non illustré).

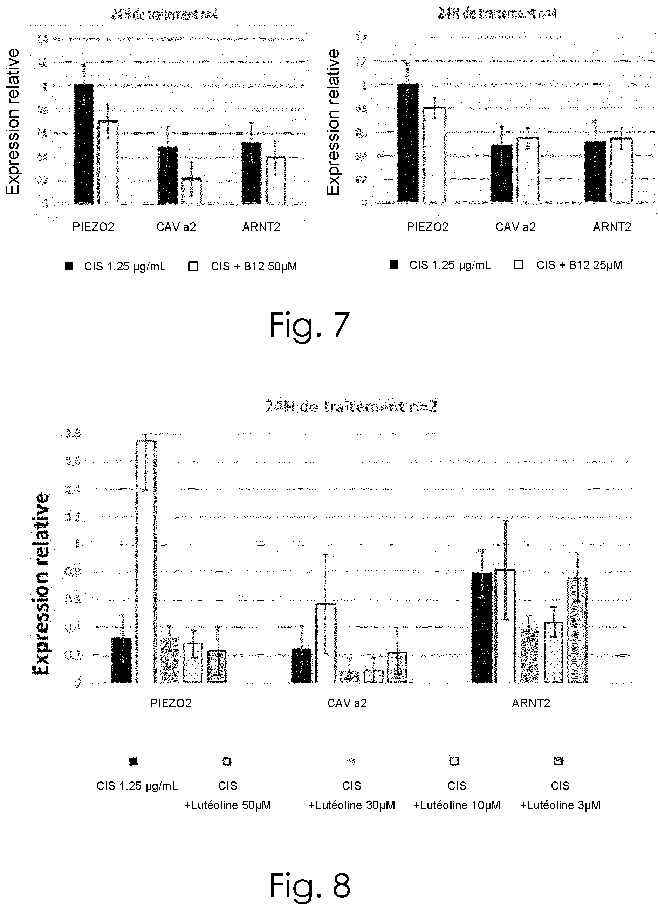

Aussi, d'après les résultats obtenus et tel qu'illustré par la Figure 7, la Vitamine B12 n'apparaît pas avoir d'effet chimio-protecteur concluant. La Vitamine B12 apparaît même avoir un peu de toxicité à une dose de 50 µM sur la morphologie neuronale (non illustré).

Enfin, aux doses utilisées, tel qu'illustré par la Figure 8, la Lutéoline n'apparaît apporter aucune amélioration ni sur l'expression des gènes ni sur la morphologie neuronale au niveau des cellules ou du réseau (non illustré).

### Exemple 5 : Test de combinaisons de molécules avec le cisplatine

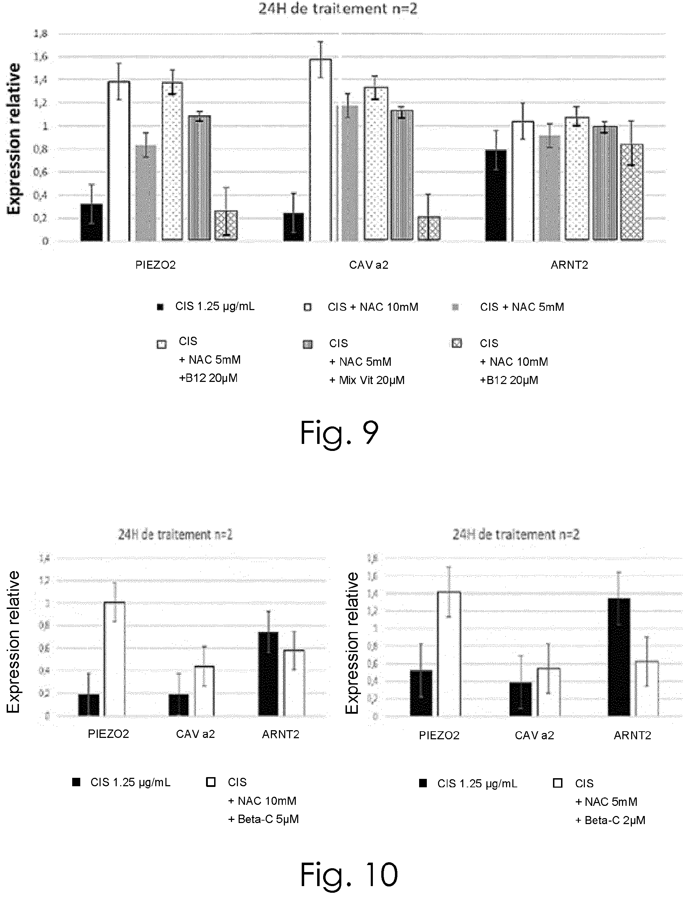

D'après les résultats obtenus et tel qu'illustré par la Figure 9, de manière particulièrement surprenante, on remarque un effet synergique de la Vitamine B12 (à une dose de 20µM) pris en combinaison avec la NAC qui font qu'à une dose de 5mM de NAC + Vitamine B12, la combinaison est aussi efficace quant à l'amélioration de l'expression des gènes notamment Piezo et CaVa2δ-1, qu'une dose de NAC de 10 mM prise seule, alors que la Vitamine B12 n'avait pas d'effet prise seule à une dose de 25 et 50µM (voir Figure 7), et présente un effet supérieur à une même dose de NAC (5mM) prise seule.

On note cependant qu'il apparaît y avoir un effet toxique de la combinaison NAC + Vitamine B12 (à une dose de 20µM) lorsque l'on double la concentration de NAC (10mM).

Aussi, on note une très bonne amélioration de la morphologie et des réseaux de neurones avec la combinaison NAC (5 mM) + Vitamine B12 (20µM) (non illustré).

De même, et de manière surprenante, on remarque également un effet synergique de la combinaison NAC (5mM) + mélange de Vitamines B1, B2 et B3 quant à l'amélioration de l'expression des gènes en particulier Piezo, alors que le mélange de Vitamines B1-B3 n'a pas d'effet pris seul.

En revanche, à titre comparatif, d'après les résultats obtenus et tel qu'illustré par la Figure 10, on note qu'une combinaison NAC (10 mM) et Beta Carotène (5 et 2µM) est plutôt négative par rapport aux résultats obtenus avec les mêmes molécules prises seules (voir respectivement Figures 5 et 6).

En outre, une toxicité sur la morphologie des cellules neuronales peut être observée avec la combinaison NAC + Beta Carotène de 5µM (non illustré).

### Exemple 6 : Gélule A selon l'invention

Selon un mode de réalisation préféré de l'invention, la composition se présente sous forme de gélule, par exemple d'environ 400 mg

| **Composition** | **% en poids du poids total de la composition** |
|---|---|
| NAC | 75 |
| Vitamine B12 | 0,06 |
| Excipients (xylitol, gomme laque) | 24,94 |

### Exemple 7 : Gélule B selon l'invention

Selon un autre mode de réalisation préféré de l'invention, la composition se présente sous forme de gélule, par exemple d'environ 400 mg

| **Composition** | **% en poids du poids total de la composition** |
|---|---|
| NAC | 25,02 |
| Vitamine B1, B2 et B3 (à proportions égales) | 50,04 |
| Excipients (xylitol, gomme laque) | 24,94 |

### Exemple 8: Procédé de fabrication de microgranules

### Etape 1 : Préparation de la solution de gomme-laque

Dans un gobelet doseur, contenant une quantité de gomme-laque pesée avec précision, ajouter, en agitant, une quantité suffisante d'éthanol (solvant disparaissant au cours de la fabrication) pour préparer une solution à 20 %.

Remuer jusqu'à ce que la gomme-laque soit complètement dissoute.

### Étape 2 : Application de la formule NAC et Vitamine B12 et/ou Vitamines B1-3

Insérez la quantité spécifiée de microgranules neutres dans la turbine. Activer le dispositif de rotation. L'application de la formule NAC et Vitamine B12 et/ou Vitamines B1-3 se fait par fractionnement : Pulvériser, à l'aide du pistolet, une partie de la solution alcoolique de gomme-laque et, immédiatement après, introduire, à l'aide d'une pelle, une partie de la quantité prescrite du mélange de poudres de la formule NAC et Vitamine B12 et/ou Vitamines B1-3 préparées.

Laisser sécher entre 2 pulvérisations/applications, le temps nécessaire (de 30 à 60 secondes) à température ambiante tout en maintenant la rotation.

Répéter ces étapes jusqu'à l'utilisation complète de la quantité prescrite du mélange de la formule NAC et Vitamine B12 et/ou Vitamines B1-3.

### Étape 3 : Tamisage

Tamiser les microgranules à l'aide d'une grille de diamètre approprié (1,00 à 1,18 mm). Seuls les microgranules passant à travers le tamis sont réintroduits dans la turbine pour l'opération de pelliculage.

### Etape 4 : Séchage

Sécher les microgranules dans la turbine en rotation pendant le temps nécessaire (cycle de 8 à 15 heures) à une température comprise entre 40 et 45°C.

Si nécessaire, les particules retenues sur les tamis sont retirées :
elles sont introduites dans la turbine contenant les microgranules avec une quantité suffisante de solution alcoolique de gomme-laque où elles subissent de nouveau les étapes 2 et 3.

### Étape 5 : Filage

Appliquez la solution alcoolique de gomme-laque précédemment préparée sur les microgranules, à l'aide du pistolet de pulvérisation.

### Étape 6 : Tamisage

Tamisez les microgranules à l'aide d'une grille de diamètre approprié (1,18 à 1,25 mm). Seuls les microgranules passant au travers du tamis sont réintroduits dans la turbine pour les opérations suivantes.

### Etape 7 : Séchage

Séchage des microgranules dans la turbine en rotation pendant le temps nécessaire (cycle de 8 à 15 heures) à une température inférieure à 50° C.

### Etape 8 : Revêtement

Appliquer, à l'aide du pistolet, la dispersion aqueuse de gomme laque prête à l'emploi sur les microgranules.

### Étape 9 : Tamisage

Tamiser les microgranules à l'aide d'une grille de diamètre approprié (1,18 à 1,25 mm). Seuls les microgranules passant au travers du tamis sont réintroduits dans la turbine pour les opérations suivantes.

### Étape 10 : Séchage

Sécher les microgranules dans la turbine en rotation pendant le temps nécessaire (cycle de 8 à 15 heures) à une température inférieure à 50° C.

### Etape 11 : Lubrification

Introduire la quantité spécifiée de talc (0,3 à 0,4 kg) en petites quantités. Remuer vigoureusement. Cela facilite l'étape suivante en réduisant l'électricité statique des microgranules.

### Étape 12 : Remplissage des capsules

Les capsules vides sont remplies de microgranules jusqu'à la masse de remplissage requise.

### Étape 13 : Emballage

Les capsules remplies sont emballées dans des blisters, puis dans des boîtes en carton.

## Revendications

1. Composition comprenant, dans un milieu physiologiquement acceptable, de la N-acétylcystéine (NAC), **caractérisée en ce qu'**elle comprend en outre de la Vitamine B12 à une concentration comprise entre 0,04 et 0,08% en poids du poids total de la composition et/ou une combinaison de Vitamines B1, B2 et B3, pour son utilisation dans la prévention et/ou le traitement des douleurs nociceptives et/ou neuropathiques chimio-induites.

2. Composition pour son utilisation selon la revendication 1, **caractérisée en ce que** la chimiothérapie est induite par les dérivés de platine choisis parmi le cisplatine, l'oxiplatine et le carboplatine, ou les taxanes choisis parmi le paclitaxel et le docétaxel.

3. Composition pour son utilisation selon l'une des revendications précédentes, **caractérisée en ce qu'**elle comprend la NAC à une concentration comprise entre 10 et 90% en poids du poids total de la composition.

4. Composition pour son utilisation selon la revendication 3, **caractérisée en ce qu'**elle comprend la NAC à une concentration comprise entre 25 et 75% en poids du poids total de la composition.

5. Composition pour son utilisation selon l'une des revendications précédentes, **caractérisée en ce qu'**elle comprend la Vitamine B12 à une concentration comprise entre 0,05 et 0,07% en poids du poids total de la composition.

6. Composition pour son utilisation selon l'une des revendications précédentes, **caractérisée en ce qu'**elle comprend une combinaison de Vitamines B1, B2 et B3, à des proportions différentes ou égales, à une concentration totale comprise entre 20 et 90% en poids du poids total de la composition.

7. Composition pour son utilisation selon la revendication 6, **caractérisée en ce qu'**elle comprend une combinaison de Vitamines B1, B2 et B3, à des proportions différentes ou égales, à une concentration totale comprise entre 40 et 60% en poids du poids total de la composition.

8. Composition pour son utilisation selon l'une des revendications précédentes, **caractérisée en ce qu'**elle comprend la Vitamine B12 prise seule ou en association avec la combinaison de Vitamines B1, B2 et B3.

9. Composition pour son utilisation selon l'une des revendications précédentes, **caractérisée en ce qu'**elle est sous une forme adaptée pour une administration par voie orale.

10. Composition pour son utilisation selon la revendication 9, **caractérisée en ce qu'**elle se présente sous forme de microgranules à libération contrôlée en gélule.

11. Produit comprenant :
- à titre de premier principe actif de la NAC, et
- à titre de deuxième principe actif de la vitamine B12 à une concentration comprise entre 0,04 et 0,08% en poids du poids total de la composition et/ou une combinaison de vitamines B1, B2 et B3,
comme produit de combinaison pour une utilisation simultanée, séparée ou étalée dans le temps pour son utilisation dans la prévention et/ou le traitement des douleurs nociceptives et/ou neuropathiques chimio-induites.

## Patentansprüche

1. Zusammensetzung, umfassend in einem physiologisch verträglichen Medium N-Acetylcystein (NAC), **dadurch gekennzeichnet, dass** sie ferner Vitamin B12 in einer Konzentration, die zwischen 0,04 und 0,08 Gew.-% des Gesamtgewichts der Zusammensetzung liegt und/oder eine Kombination der Vitamine B1, B2 und B3, zur Verwendung in der Vorbeugung und/oder der Behandlung von durch Chemotherapie induzierten nozizeptiven und/oder neuropathischen Beschwerden, umfasst.

2. Zusammensetzung zur Verwendung nach Anspruch 1, **dadurch gekennzeichnet, dass** die Chemotherapie durch Platinderivate, ausgewählt aus Cisplatin, Oxiplatin und Carboplatin oder Taxane, ausgewählt aus Paclitaxel und Docetaxel, induziert wird.

3. Zusammensetzung zur Verwendung nach einem der vorstehenden Ansprüche, **dadurch gekennzeichnet, dass** sie NAC in einer Konzentration, die zwischen 10 und 90 Gew.-% des Gesamtgewichts der Zusammensetzung liegt, umfasst.

4. Zusammensetzung zur Verwendung nach Anspruch 3, **dadurch gekennzeichnet, dass** sie NAC in einer Konzentration, die zwischen 25 und 75 Gew.-% des Gesamtgewichts der Zusammensetzung liegt, umfasst.

5. Zusammensetzung zur Verwendung nach einem der vorstehenden Ansprüche, **dadurch gekennzeichnet, dass** sie Vitamin B12 in einer Konzentration, die zwischen 0,05 und 0,07 Gew.-% des Gesamtgewichts der Zusammensetzung liegt, umfasst.

6. Zusammensetzung zur Verwendung nach einem der vorstehenden Ansprüche, **dadurch gekennzeichnet, dass** sie eine Kombination der Vitamine B1, B2 und B3 in unterschiedlichen oder gleichen Anteilen mit einer Gesamtkonzentration, die zwischen 20 und 90 Gew.-% des Gesamtgewichts der Zusammensetzung liegt, umfasst.

7. Zusammensetzung zur Verwendung nach Anspruch 6, **dadurch gekennzeichnet, dass** sie eine Kombination der Vitamine B1, B2 und B3 in unterschiedlichen oder gleichen Anteilen mit einer Gesamtkonzentration, die zwischen 40 und 60 Gew.-% des Gesamtgewichts der Zusammensetzung liegt, umfasst.

8. Zusammensetzung zur Verwendung nach einem der vorstehenden Ansprüche, **dadurch gekennzeichnet, dass** sie Vitamin B12, das allein oder in Verbindung mit den Vitaminen B1, B2 und B3 eingenommen wird, umfasst.

9. Zusammensetzung zur Verwendung nach einem der vorstehenden Ansprüche, **dadurch gekennzeichnet, dass** sie eine Form aufweist, die für eine Verabreichung auf oralem Weg angepasst ist.

10. Zusammensetzung zur Verwendung nach Anspruch 9, **dadurch gekennzeichnet, dass** sie in Form von Mikrogranulat mit kontrollierter Freisetzung in Kapseln vorliegt.

11. Produkt umfassend:
- als ersten Wirkstoff NAC und
- als zweiten Wirkstoff Vitamin B12 in einer Konzentration, die zwischen 0,04 und 0,08 Gew.-% des Gesamtgewichts der Zusammensetzung liegt, und/oder einer Kombination der Vitamine B1, B2 und B3,
als Kombinationspräparat für eine gleichzeitige, getrennte oder zeitlich verteilte Verwendung, zur Verwendung in der Vorbeugung und/oder Behandlung von durch Chemotherapie induzierten nozizeptiven und/oder neuropathischen Beschwerden.

## Claims

1. Composition comprising, in a physiologically acceptable medium, N-acetylcysteine (NAC), **characterized in that** it further comprises vitamin B12 at a concentration of between 0.04 and 0.08% by weight of the total weight of the composition and/or a combination of vitamins B1, B2 and B3, for use thereof in the prevention and/or treatment of chemo-induced nociceptive and/or neuropathic pains.

2. Composition for use thereof according to claim 1, **characterized in that** the chemotherapy is induced by platinum derivatives chosen from cisplatin, oxiplatin and carboplatin, or taxanes chosen from paclitaxel and docetaxel.

3. Composition for use thereof according to one of the preceding claims, **characterized in that** it comprises NAC at a concentration of between 10 and 90% by weight of the total weight of the composition.

4. Composition for use thereof according to claim 3, **characterized in that** it comprises NAC at a concentration of between 25 and 75% by weight of the total weight of the composition.

5. Composition for use thereof according to one of the preceding claims, **characterized in that** it comprises vitamin B12 at a concentration of between 0.05 and 0.07% by weight of the total weight of the composition.

6. Composition for use thereof according to one of the preceding claims, **characterized in that** it comprises a combination of vitamins B1, B2 and B3, in different or equal proportions, at a total concentration of between 20 and 90% by weight of the total weight of the composition.

7. Composition for use thereof according to claim 6, **characterized in that** it comprises a combination of vitamins B1, B2 and B3, in different or equal proportions, at a total concentration of between 40 and 60% by weight of the total weight of the composition.

8. Composition for use thereof according to one of the preceding claims, **characterized in that** it comprises vitamin B12, taken alone or associated with the combination of vitamins B1, B2 and B3.

9. Composition for use thereof according to one of the preceding claims, **characterized in that** it is in a form suitable for oral administration.

10. Composition for use thereof according to claim 9, **characterized in that** it is in the form of controlled-release microgranules in gel capsules.

11. Product comprising:
- NAC as first active ingredient, and
- vitamin B12 at a concentration of between 0.04 and 0.08% by weight of the total weight of the composition, and/or a combination of vitamins B1, B2 and B3, as second active ingredient,
as a combination product for use simultaneously, separately or staggered over time in the prevention and/or treatment of chemo-induced nociceptive and/or neuropathic pains.
